**Europäisches Patentamt**

**European Patent Office**

**Office européen des brevets**

(11) Publication number : **0 337 398 B1**

# EUROPEAN PATENT SPECIFICATION

(45) Date of publication of patent specification :
**26.08.92 Bulletin 92/35**

(51) Int. Cl.⁵ : **A61K 35/78**

(21) Application number : **89106422.2**

(22) Date of filing : **11.04.89**

(54) **Methods and compositions for the treatment of NIDDM.**

The file contains technical information
submitted after the application was filed and
not included in this specification

(73) Proprietor : **BATTELLE MEMORIAL INSTITUTE**
**505 King Avenue**
**Columbus Ohio 43201-2693 (US)**

(30) Priority : **12.04.88 US 180420**

(72) Inventor : **Wilson, Bary W.**
**34011 Caballo Road**
**Kennewick Washington 99337 (US)**
Inventor : **Campbell, James A.**
**5504 West Melville**
**Pasco Washington 99301 (US)**
Inventor : **Sasser, Lyle B.**
**1805 McPherson**
**Richland Washington 99352 (US)**
Inventor : **Hortal, Patricio**
**Rawson 2557**
**Mar del Plata 7600 (AR)**

(43) Date of publication of application :
**18.10.89 Bulletin 89/42**

(45) Publication of the grant of the patent :
**26.08.92 Bulletin 92/35**

(84) Designated Contracting States :
**AT BE CH DE ES FR GB GR IT LI LU NL SE**

(74) Representative : **Brown, John David et al**
**FORRESTER & BOEHMERT**
**Franz-Joseph-Strasse 38**
**W-8000 München 40 (DE)**

(56) References cited :
**No relevant documents have been disclosed**

## Description

Technical Field

The present invention is generally directed toward compositions for use in the treatment of maturity onset on-insulin dependent diabetes mellitus (NIDDM), and more specifically, to the management of NIDDM through the use of hypoglycemic agents.

Background of the Invention

Maturity onset non-insulin dependent diabetes mellitus (NIDDM) and associated preclinical conditions, such as insufficient glucose tolerance (IGT), are characterized by the diminished capacity of islet cells of the pancreas to synthesize and release sufficient insulin in response to rising blood glucose levels. Decreased sensitivity to insulin in peripheral tissues of many diabetics is also observed. New case reports of NIDDM and IGT appear to be increasing at an accelerating rate in several Western countries (Zimmet and King, World Trends in Diabetes Epidemiology, 1986). In the United States, for example, it is estimated that approximately five percent of the adult population suffers from some form of diabetic condition, with the rate of new cases increasing at about six percent per year, or approximately 600,000 new cases each year. An additional, not well-defined population exhibits preclinical symptoms such as IGT.

The management of NIDDM requires special dietary measures, and often the use of a pharmaceutical hypoglycemic agent. These agents have the ability to stimulate insulin production in the islet cells and, in conjunction with dietary measures, can help to stabilize blood sugar levels.

There are currently two chemically distinct families of oral hypoglycemic agents used in the management of NIDDM. One such family of agents, the biguanides, have been largely withdrawn from the commercial market in the United States because they have been associated with rare, but sometimes fatal, side effects. The other major family, sulfonylureas, are presently sold under approximately 100 different brand names, and as a family, constitute the only oral hypoglycemic agent in widespread use at this time in the United States. Sulfonylureas, however, fail to control hyperglycemia on initial use in approximately 30 percent to 40 percent of new cases (primary failures), and in an additional 1 to 5 percent of the new cases, they eventually lose their effectiveness (secondary failures).

There are also a variety of natural products which appear to exhibit hypoglycemic activity. These products are generally plants or plant-derived compounds, usually in the form of a somewhat crude extract. It is estimated that more than 200 species of plants exhibit hypoglycemic properties, including many common plants, such as immature bean pods, olive leaves, potatoes, wheat, celery, blackberry leaves, sugar beets, and the leaves and roots of bananas (Farmsworth and Segelman, Tile Till 57:52-55, 1971).

Other potential hypoglycemic agents have been isolated, for example, from the leaves of Aloe Aboraescens Var Natalis (Hikino et al., Int. J. Crude Drug Res. 24:183-186, 1986) and from the roots of Oryza-Sativa (Hikino et al., Planta Med. 0:490-492, 1986). In addition, studies in India have shown that leaf extracts of Gymnema sylvestre can prolong the lifespan of Alloxan-induced diabetic rats. However, use of these extracts in intact animals leads to highly variable results. These extracts also induce hypoglycemia in the Alloxan model (Srivastava et al., Int. J. Crude Drug Res. 24:171-176, 1986).

The seeds of Eugenia jambolana, another plant found in India, appear to exhibit hypoglycemic activity comparable to that of chlorpropamide, as determined by effects on cathepsin B (Bansal et al., Indian J. Biochem. Biophy. 18:377, 1981). Further, it has been reported that Salvia lavandulifolia possesses a slight hypoglycemic activity that is independent of the effects of insulin (Jimenez et al., Planta Med. 1:260-262, 1986). Other folklore remedies, including tea made from herbs such as Allofylus edulis (Barboza et al., Plantas Que Curan, 1985), Daucus carota and Catharanthus roseus, have been sold for the control of diabetes in South American or Southeast Asian countries.

Due to the lack of consistent clinical effectiveness for previously suggested synthetic oral hypoglycemic agents, there is a need in the art for additional, safe, oral hypoglycemic agents that provide the clinician with a wider range of options in managing maturity onset NIDDM. The present invention fills this need, and further provides other related advantages.

Summary of the Invention

Briefly stated, the present invention provides a variety of hypoglycemic compositions for use in the maintenance of maturity onset non-insulin dependent diabetes mellitus (NIDDM). In one aspect of the present invention, the hypoglycemic composition comprises a polar solvent extract of at least one part of the plant

_Hexachlymus_ _edulis_ or related _Hexachlymus_ species. The part may include the leaves, seed pods, roots or bark of the plant. Within a preferred embodiment, the polar solvent extract is an alkanol or aqueous alkanol extract. Particularly preferred is an aqueous ethanol extract.

The compositions of the present invention are also useful in a method for reducing the blood glucose level in a patient, comprising administering to the patient an effective amount of a composition comprising a polar solvent extract of at least one part of the plant _Hexachlymus_ _edulis_ or related _Hexachlymus_ species, in combination with a physiologically acceptable carrier or diluent. Suitable carriers or diluents include alcohol, water, physiological saline, dimethyl sulfoxide, and mixtures thereof.

A method for preparing the hypoglycemic compositions briefly described above is also provided. The method comprises (a) macerating at least one part of the plant _Hexachlymus_ _edulis_, or related _Hexachlymus_ species, with a polar solvent to yield a solution and solid plant debris; and (b) separating the solution from the solid plant debris, thereby yielding an extract suitable as a hypoglycemic composition.

The compositions of the present invention are useful within a method for reducing symptomatic conditions associated with diabetes in a patient, such as polydipsia, polyuria and polyphagia. The method comprises administering to the patient an effective amount of a composition comprising a polar solvent extract of at least one part of the plant _Hexachlymus_ _edulis_ or related _Hexachlymus_ species, in combination with a physiologically acceptable carrier or diluent.

These and other aspects of the present invention will become evident upon reference to the following detailed description and attached drawing.

Brief Description of the Drawing

The figure shows a 12 month clinical history of a patient receiving a representative polar solvent leaf alcohol extract of the present invention.

Detailed Description of the Invention

As noted above, the present invention is directed toward the use of certain hypoglycemic compositions, such as a polar solvent extract of at least one portion of the plant _Hexachlymus_ _edulis_ (_H._ _edulis_) or related _Hexachlymus_ species. Because there are a number of common names for _H._ _edulis_ and they vary according to the region and language spoken, "_H._ _edulis_" as used herein includes all common names, such as Yva'hai, Ibajai and Cereja do Rio Grande.

_H._ _edulis_ and related _Hexachlymus_ species are bush-like plants which reach the size of a small tree when fully grown. They grow between 5 and 12 meters high with a dap of 25 to 70 centimeters. The tree-top is globose with abundant, tortuous ramification, while the trunk is straight and cylindrical. The fust or main shaft is 2 to 6 meters in length. The plants are heliotropes, which grow abundantly in the eastern region of Paraguay as well as along the Paraguayan River and the Parana River. They are also found in humid areas along the jungle border and in other open areas.

The plant can be generally identified by the following characteristics: (a) leaves that grow in opposite pairs, elliptical in shape and aromatic with translucent tips; (b) external bark which is dark brown in color with longitudinal groves forming short, rib-like lines; (c) inner bark which is a light pink color; and (d) fruit which is spherical, yellow, the outer skin of which usually contains fuzz and has an unpleasant odor. The following is a more specific botanical description of _H._ _edulis_:

Bark--the outer bark is rough, hard, with longitudinal grooves forming short, rib-like lines. The color is dark brown. The bark is 3 to 6 millimeters thick. The inner bark's color varies from light pink to light brown, with the color changing to dark red at the outer edges. The inner bark is 10 to 20 millimeters thick.

Leaves--the leaves grow in opposite pairs and their shape is elliptical, ranging from between 3 to 8 centimeters long and 1 to 3 centimeters wide. The leaves also have long, translucent tips and smooth, rounded edges. The young leaves have fuzz which disappears with age. When the leaves are crushed, they emit an unpleasant odor characteristic of the plant.

Flowers and Fruit--the flowers are singular, white, 1.5 to 2.0 centimeters wide, with five petals and numerous stamen. The fruit is categorized as a drupe, subglobose in shape and yellow in color with fuzz. The fruit is between 3 to 6 centimeters in diameter, with five sharp-pointed fuzzy sepals which are attached at the apex. The fruit is edible, but very sour and acidic. There are one to two seeds in each fruit. The plants generally flower during June and September and fructify between September and November.

A hypoglycemic composition may be prepared from one or more parts, i.e., leaves, seed pods, roots or bark, of _H._ _edulis_ or related _Hexachlymus_ species. Although dry-stored, but not desiccated, leaves may be used, fresh leaves are preferred. A suitable method for preparing such a composition includes macerating at least one part of the plant with a polar solvent, preferably at room temperature and with occasional stirring, for from 1 hour to 45 days to yield a highly colored solution and solid plant debris. A 7-day time period for the extraction is preferred. In this regard, it should be noted that longer maceration times may yield more potent activity for the extract. Following maceration, although not essential, it is preferable to separate the solution from the solid plant debris. For example, the solution may be filtered to effectuate removal of the solid plant debris.

In general, and as partially discussed above, suitable polar solvent extracts may be prepared utilizing a number of methods that result in the basic separation of the constituents of interest from the remaining plant material. Suitable polar solvents include alkanols, phenols, acetonitrile, tetrahydrofuran, chlorinated hydrocarbons, and aqueous mixtures thereof. A preferred polar solvent is an alkanol or aqueous mixture thereof ("aqueous alkanol"). As used herein, "alkanols" include straight-chain and branched-chain alkyl alcohols, such as methanol, ethanol, propanol, and isopropanol. Particularly preferred is aqueous ethanol. Alkanol extracts from the seed pods and root materials appear to be more potent than similar leaf extracts.

Prior to the step of macerating, it may be desirable to physically disrupt the part of the plant that is utilized. It will be evident to one skilled in the art that there are a variety of ways in which the plant may be prepared prior to extraction as described within the present invention. For instance, the leaves, seed pods, roots or bark may be chopped, crushed or ground to increase the surface area exposed to the polar solvent.

The compositions of the present invention may be administered to patients in a variety of forms and by a variety of routes. Irrespective of the form of the composition and the route of the administration, it is preferable that a regular dosage regime, i.e., daily, be maintained, as well as an appropriate diet

As noted above, the compositions according to the invention are useful within method for reducing the blood glucose level in a patient that comprises administering to the patient an effective amount of a composition comprising a polar solvent extract of at least one part of the plant Hexachlymus edulis or related Hexachlymus species, in combination with a physiologically acceptable carrier or diluent. Physiologically acceptable carriers and diluents include alcohols, water, physiological saline, dimethyl sulfoxide (DMSO), and mixtures thereof. The polar solvent extract may be administered by a variety of routes, including orally and transdermally. For oral administration, aqueous alcohol is a preferred diluent, and aqueous ethanol is particularly preferred. For transdermal administration, DMSO is a preferred carrier.

The effective and safe dosage range in humans appears to be wide. In clinical studies with 39 patients having maturity onset diabetes, no episodes of hypoglycemia were reported. Oral dosages of a polar solvent leaf extract that have been effective in lowering blood glucose levels in humans without hypoglycemic side effects range typically from about 0.25 ml to about 15 ml of the polar solvent leaf extract which has been diluted 10-fold with water or physiological saline. These dosages were administered daily. A preferred dosage of the polar solvent leaf extract is about 1.5 ml to 5 ml per day. Daily oral dosages of a polar solvent seed extract range typically from about 0.1 ml to about 5 ml when diluted 10-fold with water or physiological saline. A preferred dosage of the polar solvent seed extract is about 0.5 ml to 2 ml per day. On a dry weight basis, these dosages are estimated to range from approximately 10 milligrams to several hundred milligrams per day. Higher dosages may be indicated for extracts in cases of very high blood glucose levels (300 mg/dl range). By way of comparison, dosages of Diabenise® (chlorpropamide) are generally held in the 100250 mg per day range to avoid hypoglycemic side effects.

In healthy rats, the dosage range (in milligrams per kilogram body weight) required to effect a 30% decrease in blood glucose levels appeared to be higher than that required to lower glucose levels in hyperglycemic humans. Rats were treated with daily dosages as high as 80 mg/kg body weight (administered twice daily) to effect the 30% decrease, whereas human dosages were in the 1 to 20 mg/kg body weight range. By way of comparison, chlorpropamide dosages are in the 1 to 3 mg/kg body weight range for humans.

Treatment of patients with the aqueous ethanol extract has resulted in the successful management of NIDDM, even where other oral hypoglycemic agents have failed. For instance, the aqueous ethanol extract succeeded in several patients where sulfonylureas, such as chlorpropamide, had failed.

The compositions according to the invention are useful within a method for reducing symptomatic conditions associated with diabetes in a patient. Symptomatic conditions include polydipsia, polyuria and polyphagia. The compositions described within the present invention, when administered to a patient, appeared to effectuate a reduction in the symptomatic conditions associated with diabetes prior to a reduction in blood glucose levels.

One method for reducing symptomatic conditions comprises administering to the patient an effective amount of a composition comprising a polar solvent extract of at least one part of the plant Hexachlymus edulis or related Hexachlymus species, in combination with a physiologically acceptable carrier or diluent. Preferred parts of the plant include leaves, seed pods, roots and bark. Suitable polar solvents include those described above. A preferred polar solvent is an alkanol or aqueous mixture thereof. Particularly preferred is aqueous ethanol. Suitable routes of administration and acceptable carriers and diluents are described above. Typical and preferred dosages, as well as suitable concentrations, are also described above.

The following examples are offered by way of illustration, and not by way of limitation.

## EXAMPLE I

### A. Method of Preparation for Aqueous Ethanol Tincture

Aqueous ethanol extracts used in the clinical studies described herein were prepared by mixing 200 grams of finely ground H. edulis leaves with 1 liter of 35% alcohol in a tightly closed glass bottle. The mixture was allowed to stand for 15 days, the bottle shaken for 3 minutes once each day. On the 15th day, an additional 1 liter of 35% alcohol was added, bringing the total to 2 liters. The tightly closed bottle was then allowed to macerate for an additional 30 days, during which the bottle was shaken for 3 minutes each day. After a total of 45 days, the mixture was strained and allowed to slowly percolate 20 drops per minute for a minimum of 10 hours. The same method was used to prepare the active seed and root tinctures.

For human consumption, a water/alcohol solution of 1 part distilled water and 1 part alcohol (35%) was prepared. The final mixture was 1 part of the tincture extract to 9 parts of the water/alcohol solution (10-fold dilution).

### B. Administration of Diluted Aqueous Ethanol Tincture

Patients were given between 20 to 30 drops (about 0.05 ml per drop) of the diluted aqueous ethanol tincture, administered under the tongue, 3 times per day, depending on the severity of their diabetic condition, one-half hour before each meal.

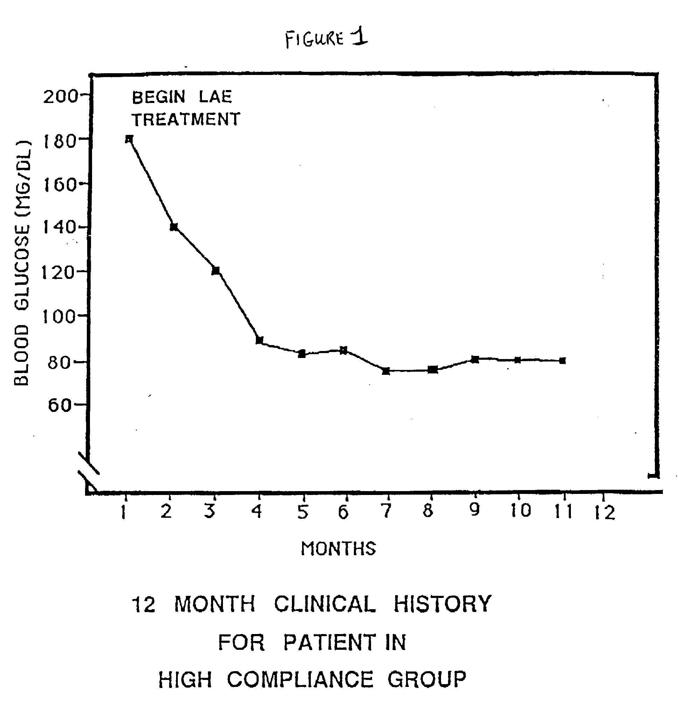

Figure 1 is a 12 month clinical history of a patient treated with a polar solvent leaf alcohol extract (LAE). The blood glucose level declines to a level within the normal range at which point it stabilizes.

Table 1 shows the reduction of blood glucose levels by extract (SAE).

## TABLE 1

| Patient | Glucose Level Before SAE Treatment | Glucose Level After SAE Treatment |
|---|---|---|
| CB | 166 | 110 |
| HC | 125 | 80 |
| LC | 200 | 136 |
| MC | 180 | 134 |
| EG | 154 | 105 |
| BL | 147 | 86 |
| AM | 155 | 135 |
| EM | 148 | 108 |
| CS | 230 | 140 |
| MU | 160 | 110 |

Glucose testing method - Hexoquinasa - mg/dl.

Table 2 is a comparison of the leaf and seed alkynol extracts in the control of blood glucose levels in ten patients suffering from NIDDM.

## TABLE 2

| Patient | Before SAE Treatment (After LAE Treatment) Glucose Level | After SAE Treatment Glucose Level |
|---|---|---|
| JM | 120 | 100 |
| IM | 145 | 120 |
| HC | 160 | 135 |
| CR | 180 | 125 |
| BM | 170 | 110 |
| AR | 110 | 100 |
| FG | 145 | 118 |
| JS | 130 | 90 |
| JB | 148 | 127 |
| MG | 134 | 87 |

Glucose testing method used - hexoquinasa in mg/dl

The H. Edulis polar solvent seed alcohol extract (SAE) proved to be 20% more effective in decreasing blood sugar levels as compared to results under the same conditions using the polar solvent leaf alcohol extract (LAE). There were no failures in the study. Based on the study, the SAE is estimated to be 5 to 10 times more potent than the LAE.

A study was conducted wherein ten patients who had been on the H. edulis extract treatment for several months were divided into two groups of five each. One group was given a placebo, and the second group was retained on the H. edulis extract. Placebo patients were not informed, and were not aware of the change in their treatment. Within 3-4 days all of the placebo patients reported the return of diabetic symptoms such as polyuria.

## TABLE 3

Blood glucose measurements in diabetic patients immediately before crossover and at approximately 7 days after crossover.

| | Average Blood Glucose Levels (mg/dl) | |
|---|---|---|
| | Before Crossover | At 7 Days |
| Placebo Group | 108 +/- 13 | 154 +/- 11 |
| Treatment Group | 103 +/- 11 | 103 +/- 11 |

Patients receiving the placebo experienced an approximate 43% increase in fasting blood glucose levels (see Table 3). Patients who remained on the extract showed no change in their average blood glucose levels at 7 days after crossover. None of these latter patients reported any diabetic symptoms during the study.

C. Summary of Human Clinical Results

Thirty-nine patients were treated with either the LAE or SAE extracts according to the following protocol. The human subjects in the studies were volunteers, and represent a cross section with regard to age, sex and clinical outlook. Each patient served as his or her own control, i.e., blood glucose levels were determined before and after treatment. Patients diagnosed as needing the agent were at or above the 180 mg/dl blood glucose level while fasting. Each patient was given the same amount of tea twice per day. All 39 patients were able to achieve and maintain preclinical blood glucose levels within 30 days. Polyurea, polyphagia, and polydipsia symptoms, when present, were alleviated within 1 to 3 days with SAE and within 7-15 days with LAE. Of these patients, at least 5 had previously been taking chlorpropamide, but were not able to attain preclinical blood sugar levels with this hypoglycemic agent. There were no incidents of hypoglycemia and no primary or secondary failures using the LAE and SAE extracts over the 4 year course of the studies.

EXAMPLE II

Comparative Animal Studies

Both healthy and genetically diabetic mice, as well as healthy rats, have shown statistically significant decreases in blood glucose levels after treatment with an H. edulis extract. In these studies, both acute and sustained effects on blood glucose have been observed.

In one animal study, 3 preparations were evaluated. The test fractions were identified as (1) control, (2) chlorpropamide, and (3) an aqueous ethanol extract. Forty rats were divided into 3 treatment groups and were orally gavaged with 4-5 ml of the test materials twice each day. The blood glucose levels were measured after 1 and 2 weeks of study and are shown in Table 4.

## TABLE 4
### Blood Glucose Levels in Rat Studies

#### Blood glucose (mg %)

| Sample | 7 day | 15 day |
|---|---|---|
| control | 93.7 | 111 |
| chlorpropamide | 60.1 | 56.5 |
| aqueous ethanol extract | 72.7 | 74.6 |

In a study using the db/db (diabetic) variant of the C-57 black mouse, 30 animals were divided into three groups of ten each. After fasting for 12 hours, Group 1 was gavaged with water, Group 2 with an aqueous solution containing 1 mg/ml of chlorpropamide and Group 3 with an H. edulis extract. Blood glucose levels were determined immediately prior to gavage and at one hour after gavage. Differences in levels induced by the extract were statistically significant by non-parametric tests.

Table 5 shows mean blood glucose levels for each of the groups before and after gavage.

7

## TABLE 5

### Blood glucose levels of db/db mice before and one hour after gavage.

#### Blood Glucose Levels (mg/dl)

|  | Pre-Gavage | 1-Hour Post Gavage | Difference |
|---|---|---|---|
| Control | 390 +/- 37 | 357 +/- 36 | -33 |
| Chlorpropamide | 332 +/- 41 | 275 +/- 34 | -57 |
| H. edulis | 455 +/- 44 | 348 +/- 36 | -107 |

From the foregoing, it will be evident that, although specific embodiments of the invention have been described herein for purposes of illustration, various modifications may be made without deviating from the spirit and scope of the invention.

The features disclosed in the foregoing description, in the claims and/or in the accompanying drawings may, both, separately and in any combination thereof, be material for realising the invention in diverse forms thereof.

## Claims

1.  A composition comprising a polar solvent extract of at least one part of the plant Hexachlymus edulis or related Hexachlymus species, in combination with a physiologically acceptable carrier or diluent, for use within a method for reducing the blood glucose level in a patient.

2.  A composition comprising a polar solvent extract of at least one part of the Hexachlymus edulis or related Hexachlymus species, in combination with a physiologically acceptable carrier or diluent, for use within a method for reducing symptomatic conditions associated with diabetes in a patient.

3.  A hypoglycemic composition, comprising:
    a polar solvent extract of at least one part of the plant Hexachlymus edulis or related Hexachlymus species.

4.  The composition of any of claims 1-3 wherein said polar solvent is an alkanol or an aqueous alkanol.

5.  The composition of claim 4 wherein the alkanol is ethanol.

6.  The composition of any of claims 1-3 wherein said part of the plant is selected from the leaves, seed pods, roots and bark of H. edulis or related Hexachlymus species.

7.  The composition of claim 2 wherein the symptomatic conditions are selected from polydipsia, polyuria and polyphagia.

8.  The composition of claim 3, further including a physiologically acceptable carrier or diluent.

9.  The composition of any of claims 1, 2 or 8 wherein the carrier or diluent is selected from the group consisting of alcohol, water, physiological saline, dimethyl sulfoxide, and mixtures thereof.

10. A method for preparing a hypoglycemic composition, comprising:
    macerating at least one part of the plant Hexachlymus edulis, or related Hexachlymus species, with a polar solvent.

**EP 0 337 398 B1**

11. A method for preparing a hypoglycemic composition, comprising:
    (a) macerating at least one part of the plant Hexachlymus edulis, or related Hexachlymus species, with a polar solvent to yield a solution and solid plant debris; and
    (b) separating said solution from said solid plant debris, thereby yielding an extract suitable as a hypoglycemic composition.

12. The method of claims 10 or 11 wherein said part of the plant. is selected from the leaves, seed pods, roots and bark of H. edulis or related Hexachlymus species.

13. The method of claims 10 or 11 wherein said polar solvent is an alkanol or an aqueous alkanol.

14. The method of claim 13 wherein said alkanol is ethanol.

15. The method of claims 10 or 11 including, prior to the step of macerating, physically disrupting at least one part of the plant Hexachlymus edulis or related Hexachlymus species.


**Patentansprüche**

1. Zubereitung, umfassend ein mit polarem Lösungsmittel hergestelltes Extrakt wenigstens eines Teils der Pflanze Hexachlymus edulis oder verwandter Hexachlymus-Arten, in Kombination mit einem physiologisch annehmbaren Trägerstoff oder Verdünnungsmittel, zur Verwendung bei einem Verfahren zur Reduzierung des Blutzuckerspiegels bei einem Patienten.

2. Zubereitung, umfassend ein mit polarem Lösungsmittel hergestelltes Extrakt wenigstens eines Teils der Hexachlymus edulis oder verwandter Hexachlymus-Arten, in Kombination mit einem physiologisch annehmbaren Trägerstoff oder Verdünnungsmittel, zur Verwendung bei einem Verfahren zur Reduzierung symptomatischer, mit Diabetes in Zusammenhang stehender Zustände bei einem Patienten.

3. Hypoglykämische Zubereitung, umfassend:
    ein mit polarem Lösungs mittel hergestelltes Extrakt wenigstens eines Teils der Pflanze Hexachlymus edulis oder verwandter Hexachlymus-Arten.

4. Zubereitung nach einem der Ansprüche 1-3, dadurch gekennzeichnet, daß besagtes polares Lösungsmittel ein Alkanol oder ein wäßriges Alkanol ist.

5. Zubereitung nach Anspruch 4, dadurch gekennzeichnet, daß das Alkanol Ethanol ist.

6. Zubereitung nach einem der Ansprüche 1-3, dadurch gekennzeichnet, daß besagter Teil der Pflanze ausgewählt ist aus den Blättern, Samenhülsen, Wurzeln und Rinde von H. edulis oder verwandten Hexachlymus-Arten.

7. Zubereitung nach Anspruch 2, dadurch gekennzeichnet, daß die symptomatischen Zustände ausgewählt sind aus Polydipsie, Polyurie und Polyphagie.

8. Zubereitung nach Anspruch 3, weiter gekennzeichnet durch einen (ein) physiologisch annehmbaren (annehmbares) Trägerstoff oder Verdünnungsmittel.

9. Zubereitung nach einem der Ansprüche 1, 2 oder 8, dadurch gekennzeichnet, daß der Trägerstoff oder das Verdünnungsmittel ausgewählt ist aus der Gruppe, die aus Alkohol, Wasser, physiologischer Kochsalzlösung, Dimethylsulfoxid und Mmischungen derselben besteht.

10. Verfahren zur Herstellung einer hypoglykämischen Zubereitung, umfassend:
    Mazeration wenigstens eines Teils der Pflanze Hexachlymus edulis oder verwandter Hexachlymus-Arten mit einem polaren Lösungsmittel.

11. Verfahren zur Herstellung einer hypoglykämischen Zubereitung, umfassend:
    a) Mazeration wenigstens eines Teils der Pflanze Hexachlymus edulis oder verwandter Hexachlymus-Arten mit einem polarem Lösungsmittel, um eine Lösung und feste Pflanzenbruchstücke bereitzustellen; und

b) Abtrennen besagter Lösung von besagten festen Pflanzenbruchstücken, wodurch ein Extrakt bereitgestellt wird, der als eine hypoglykämische Zubereitung geeignet ist.

**12.** Verfahren nach den Ansprüchen 10 oder 11, dadurch gekennzeichnet, daß besagter Teil der Pflanze ausgewählt ist aus den Blättern, Samenhülsen, Wurzeln und Rinde von H. edulis oder verwandten Hexachlymus-Arten.

**13.** Verfahren nach den Ansprüchen 10 oder 11, dadurch gekennzeichnet, daß besagtes polares Lösungsmittel ein Alkanol oder ein wäßriges Alkanol ist.

**14.** Verfahren nach Anspruch 13, dadurch gekennzeichnet, daß besagtes Alkanol Ethanol ist.

**15.** Verfahren nach den Ansprüchen 10 oder 11, dadurch gekennzeichnet, daß vor dem Mazerationsschritt wenigstens ein Teil der Pflanze Hexachlymus edulis oder verwandter Hexachlymus-Arten physikalisch zerbrochen wird.

## Revendications

**1.** Composition comprenant un extrait, dans un solvant polaire, d'au moins une partie de la plante Hexachlymus edulis ou d'une espèce d'Hexachlymus apparentée, en association avec un véhicule ou diluant physiologiquement acceptable, destinée à être utilisée dans une méthode d'abaissement du taux sanguin de glucose chez un patient.

**2.** Composition comprenant un extrait, dans un solvant polaire, d'au moins une partie de la plante Hexachlymus edulis ou d'une espèce d'Hexachlymus apparentée, en association avec un véhicule ou diluant physiologiquement acceptable, destinée à être utilisée dans une méthode de réduction des troubles symptomatiques associés au diabète chez un patient.

**3.** Composition hypoglycémiante, comprenant :
un extrait, dans un solvant polaire, d'au moins une partie de la plante Hexachlymus edulis ou d'une espèce d'Hexachlymus apparentée.

**4.** Composition suivant l'une quelconque des revendications 1 à 3, dans laquelle le solvant polaire est un alcanol ou une solution aqueuse d'un alcanol.

**5.** Composition suivant la revendication 4, dans laquelle l'alcanol est l'éthanol.

**6.** Composition suivant l'une quelconque des revendications 1 à 3, dans laquelle la partie de la plante est choisie entre les feuilles, les gousses, les racines et l'écorce de H. edulis ou d'une espèce d'Hexachlymus apparentée.

**7.** Composition suivant la revendication 2, dans laquelle les troubles symptomatiques consistent en une polydipsie, une polyurie ou une polyphagie.

**8.** Composition suivant la revendication 3, comprenant en outre un véhicule ou diluant physiologiquement acceptable.

**9.** Composition suivant l'une quelconque des revendications 1, 2 et 8, dans laquelle le véhicule ou diluant est choisi dans le groupe comprenant un alcool, l'eau, le sérum physiologique, le diméthylsulfoxyde et leurs mélanges.

**10.** Procédé de préparation d'une composition hypoglycémiante, consistant :
à faire macérer au moins une partie de la plante Hexachlymus edulis, ou d'une espèce d'Hexachlymus apparentée, avec un solvant polaire.

**11.** Procédé de préparation d'une composition hypoglycémiante, consistant :
(a) à faire macérer au moins une partie de la plante Hexachlymus edulis, ou d'une espèce d'Hexachlymus apparentée, avec un solvant polaire, ce qui donne une solution et des débris végétaux solides ; et
(b) à séparer ladite solution desdits débris végétaux solides, ce qui donne un extrait convenant comme composition hypoglycémiante.

**12.** Procédé suivant la revendication 10 ou 11, dans lequel la partie de la plante est choisie entre les feuilles, les gousses, les racines et l'écorce de H. edulis ou d'une espèce d'Hexachlymus apparentée.

**13.** Procédé suivant la revendication 10 ou 11, dans lequel le solvant polaire est un alcanol ou une solution aqueuse d'un alcanol.

**14.** Procédé suivant la revendication 13, dans lequel l'alcanol est l'éthanol.

**15.** Procédé suivant la revendication 10 ou 11, comprenant, avant l'étape de macération, une étape consistant à provoquer la rupture physique d'au moins une partie de la plante Hexachlymus edulis ou d'une espèce d'Hexachlymus apparentée.

FIGURE 1

12 MONTH CLINICAL HISTORY
FOR PATIENT IN
HIGH COMPLIANCE GROUP